# EUROPÄISCHE PATENTANMELDUNG

(11) **EP 0 523 513 A1**
(43) Veröffentlichungstag der Anmeldung: **20.01.1993**
(21) Anmeldenummer: 92111455.9
(22) Anmeldetag: 06.07.1992
(51) Int. Cl.: A61K 31/50

(54) **Verwendung substituierter Pyridazine zur Behandlung von Dermatosen**

(30) Priorität: 05.07.1991 CH 1998/91
(71) Anmelder: Byk Gulden Lomberg Chemische Fabrik GmbH, D-78403 Konstanz (DE)
(72) Erfinder: Schudt, Christian, Dr., W-7750 Konstanz (DE); Amschler, Hermann, Dr., W-7760 Radolfzell (DE); Flockerzi, Dieter, Dr., W-7753 Allensbach (DE); Beume, Rolf, Dr., W-7750 Konstanz 18 (DE); Riedel, Richard, Dr., W-7967 Bad Waldsee-Reute (DE)

(57) **Zusammenfassung**

Die Erfindunng betrifft die Verwendung von Verbindungen der Formel I 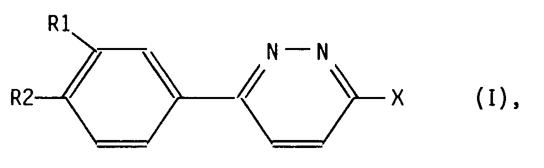
worin einer der Substituenten R1 und R2 Wasserstoff, 1-5C-Alkoxy oder Difluormethoxy, und der andere 1-5C-Alkoxy, 4-7C-Cycloalkoxy, 3-7C-Cycloalkylmethoxy, 3-5C-Alkenyloxy oder 1-4C-Polyfluoralkoxy bedeutet, X Hydroxy oder Cyanamino bedeutet, und ihren pharmakologisch verträglichen Salzen mit Basen zur Herstellung von Arzneimitteln für die Behandlung von Dermatosen.

## Beschreibung

### Technisches Gebiet

Die Erfindung betrifft die Verwendung bekannter Pyridazine zur Herstellung neuer Arzneimittel.

### Stand der Technik

In einer Reihe von Patentanmeldungen und Patenten wird die Eignung von Verbindungen verschiedener Substanzklassen zur Behandlung proliferativer Hauterkrankungen beschrieben. So sollen sich beispielsweise bestimme Xanthinderivate (siehe z.B. EP 389 282, EP 267 676, EP 260 127, WO 87/04435 und EP 195 496) und bestimmte Imidazo-chinazolinderivate (siehe z.B. US 4,837,239, US 4,690,925, US 4,663,320, EP 212 647, EP 153 152 und EP 116 948) zur Behandlung entzündlicher Hauterkrankungen eignen. - Aus den europäischen Patentanmeldungen bzw. Patenten EP 125 636, EP 163 965 und EP 393 500 sind Verbindungen bekannt, die sich aufgrund ihrer bronchospasmolytischen und cardiotonischen Wirkung zur Behandlung von Atemwegs- und Herzerkrankungen eignen sollen.

### Beschreibung der Erfindung

Es wurde nun gefunden, daß die unten näher beschriebenen Verbindungen der Formel I zur Behandlung von Dermatosen in hervorragender Weise geeignet sind.

Gegenstand der Erfindung ist die Verwendung von Verbindungen der Formel I 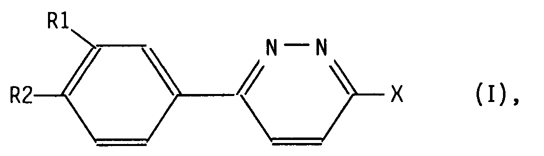
worin einer der Substituenten R1 und R2 Wasserstoff, 1-5C-Alkoxy oder Difluormethoxy, und der andere 1-5C-Alkoxy, 4-7C-Cycloalkoxy, 3-7C-Cycloalkylmethoxy, 3-5C-Alkenyloxy oder 1-4C-Polyfluoralkoxy bedeutet, X Hydroxy oder Cyanamino bedeutet, und ihren pharmakologisch verträglichen Salzen mit Basen zur Herstellung von Arzneimitteln für die Behandlung von Dermatosen.

1-5C-Alkoxy ist geradkettig oder verzweigt. Als beispielhafte 1-5C-Alkoxyreste seien genannt der Methoxy-, Ethoxy-, n-Propoxy-, Isopropoxy-, n-Butoxy-, Isobutoxy-, sec.-Butoxy-, tert-Butoxy-, n-Pentyloxy, Isopentyloxy- und der 2,2-Dimethylpropoxyrest.

4-7C-Cycloalkoxy steht beispielsweise für Cyclobutyloxy, Cyclopentyloxy, Cyclohexyloxy und Cycloheptyloxy, wovon Cyclopentyloxy bevorzugt ist.

3-7C-Cycloalkylmethoxy steht beispielsweise für Cyclopropylmethoxy, Cyclobutylmethoxy, Cyclopentylmethoxy, Cyclohexylmethoxy und Cycloheptylmethoxy, wovon Cyclopropylmethoxy und Cyclobutylmethoxy bevorzugt sind.

3-5C-Alkenyloxy ist geradkettig oder verzweigt. Die Doppelbindung von Alkenyloxy geht nicht von dem Kohlenstoffatom aus, das an das Sauerstoffatom bindet. Als beispielhafte 3-5C-Alkenyloxyreste seien genannt der Buten-2-yloxy-, der Allyloxy- und der Methallyloxyrest.

1-5C-Alkoxy ist gegenüber 3-5C-Alkenyloxy bevorzugt.

Unter 1-4C-Polyfluoralkoxy wird geradkettiges oder verzweigtes 1-4C-Alkoxy verstanden, bei dem mindestens 2 Wasserstoffatome durch Fluor ersetzt sind. Geradkettiges 1-3C-Alkoxy, bei dem mindestens 2 Wasserstoffatome durch Fluor ersetzt sind, ist bevorzugt. Bevorzugte 1-4C-Polyfluoralkoxygruppen sind Trifluormethoxy, 1,1,2,2-Tetrafluorethoxy und insbesondere Difluormethoxy und 2,2,2-Trifluorethoxy.

Unter Cyanamino wird die Gruppe -NHCN verstanden.

Als Salze kommen alle pharmakologisch verträglichen Salze der in der Galenik üblicherweise verwendeten anorganisch und organischen Basen in Betracht.

Als solche eignen sich beispielsweise wasserlösliche und wasserunlösliche Salze, wobei als Kationen für die Salzbildung vor allem die Kationen der Alkalimetalle oder Erdalkalimetalle verwendet werden; es kommen jedoch auch die entsprechenden Kationen organischer Stickstoffbasen, wie Amine oder Aminoalkanole, Aminozucker etc. zur Anwendung. Beispielsweise seien die Salze von Natrium, Magnesium, Calcium, Dimethylamin, Diethylamin, Ethanolamin, Diethanolamin, Triethanolamin, Glucamin, N-Methylglucamin (Meglumin), Glucosamin und N-Methylglucosamin genannt.

Als Dermatosen seien insbesondere proliferative, entzündliche und allergische Hauterkrankungen erwähnt. So können die Verbindungen der Formel I beispielsweise zur Verhütung und Behandlung folgender Hauterkrankungen eingesetzt werden: Psoriasis vulgaris, toxisches und allergisches Kontaktekzem, atopisches Ekzem, seborrhoisches Ekzem, follikuläre und flächenhafte Pyodermien, endogene und exogene Akne, Akne rosacea sowie andere proliferative, entzündliche und allergische Hauterkrankungen. Weiterer Gegenstand der Erfindung ist somit die Verwendung von Verbindungen der Formel I und ihren pharmakologisch verträglichen Salzen zur Behandlung von solchen Individuen, die an Dermatosen erkrankt sind.

Die Anwendung der Verbindungen der Formel I erfolgt insbesondere in Form solcher Arzneimittel, die für eine topische Applikation geeignet sind. Für die Herstellung der Arzneimittel werden die Verbindungen der Formel I und/oder ihre pharmakologisch verträglichen Salze (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt, in denen der Wirkstoffgehalt vorteilhafterweise zwischen 0,1 und 99 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Eine Gruppe (Gruppe 1) von erfindungsgemäß zu verwendenden Verbindungen sind solche der Formel I, worin einer der Substituenten R1 oder R2 Methoxy und der andere 1-5C-Alkoxy oder 3-5C-Alkenyloxy und X Hydroxy bedeutet, und ihre pharmakologisch verträglichen Salze mit Basen.

Eine Ausgestaltung der Gruppe 1 (Ausgestaltung 1a) sind Verbindungen der Formel I, worin
- R1: Methoxy,
- R2: 2-4C-Alkoxy oder 3-4C-Alkenyloxy und
- X: Hydroxy bedeutet,
und ihre pharmakologisch verträglichen Salze mit Basen.

Eine weitere Ausgestaltung der Gruppe 1 (Ausgestaltung 1b) sind Verbindungen der Formel I, worin
- R1: 2-4C-Alkoxy oder 3-4C-Alkenyloxy,
- R2: Methoxy und
- X: Hydroxy bedeutet,
und ihre pharmakologisch verträglichen Salze mit Basen.

Bevorzugte Vertreter der Ausgestaltung 1a sind solche der Formel I, worin R2 n-Propoxy, Isopropoxy oder Isobutoxy bedeutet. Besonders bevorzugte Vertreter sind die Verbindungen 6-(3-Methoxy-4-n-propoxyphenyl)-3[2H]pyridazinon und 6-(4-Isobutoxy-3-methoxyphenyl)-3[2H]-pyridazinon.

Bevorzugte Vertreter der Ausgestaltung 1b sind solche der Formel I, worin R1 n-Propoxy, Isopropoxy, Allyloxy oder Isobutoxy bedeutet. Besonders bevorzugte Vertreter sind die Verbindungen 6-(3-Isobutoxy-4-methoxyphenyl)-3[2H]pyridazinon und 6-(4-Methoxy-3n-propoxyphenyl)-3[2H]pyridazinon.

Eine weitere Gruppe (Gruppe 2) von erfindungsgemäß zu verwendenden Verbindungen sind solche der Formel I, worin einer der Substituenten R1 und R2 Wasserstoff oder 1-5C-Alkoxy und der andere 1-4C-Polyfluoralkoxy und X Hydroxy bedeutet, und ihre pharmakologisch verträglichen Salze mit Basen.

Hervorzuhebende Vertreter der Gruppe 2 sind solche der Formel I, worin einer der Substituenten R1 und R2 Wasserstoff oder geradkettiges 1-3C-Alkoxy und der andere Polyfluor-1-2C-alkoxy und X Hydroxy bedeutet, und ihre pharmakologisch verträglichen Salze mit Basen.

Bevorzugte Vertreter der Gruppe 2 sind solche der Formel I, worin einer der Substituenten R1 und R2 Wasserstoff oder Methoxy und der andere Difluormethoxy, Trifluormethoxy, 1,1,2,2-Tetrafluorethoxy oder 2,2,2,-Trifluorethoxy und X Hydroxy bedeutet, und ihre pharmakologisch verträglichen Salze mit Basen.

Ein besonders bevorzugter Vertreter der Gruppe 2 ist die Verbindung 6-(4-Difluormethoxy-3-methoxyphenyl)-3[2H]pyridazinon und ihre pharmakologisch verträglichen Salze mit Basen.

Eine weitere Gruppe (Gruppe 3) von erfindungsgemäß zu verwendenden Verbindungen sind solche der Formel I, worin einer der Substituenten R1 und R2 Methoxy, Difluormethoxy oder Ethoxy, und der andere 1-5C-Alkoxy, 4-7C-Cycloalkoxy, 3-7C-Cycloalkylmethoxy, 3-5C-Alkenyloxy oder 1-4C-Polyfluoralkoxy und X Cyanamino bedeutet, und ihre pharmakologisch verträglichen Salze mit Basen.

Eine Ausgestaltung der Gruppe 3 (Ausgestaltung 3a) sind Verbindungen der Formel I, worin
- R1: Methoxy, Difluormethoxy oder Ethoxy,
- R2: 1-4C-Alkoxy, 4-6C-Cycloalkoxy, 3-6C-Cycloalkylmethoxy, 3-4C-Alkenyloxy oder 1-2C-Polyfluoralkoxy und
- X: Cyanamino bedeuten,
und ihre pharmakologisch verträglichen Salze mit Basen.

Eine weitere Ausgestaltung der Gruppe 3 (Ausgestaltung 3b) sind Verbindungen der Formel I, worin
- R1: 2-4C-Alkoxy, 4-6C-Cycloalkoxy, 3-6C-Cycloalkylmethoxy, 3-4C-Alkenyloxy oder 1-2C-Polyfluoralkoxy,
- R2: Methoxy, Difluormethoxy oder Ethoxy und
- X: Cyanamino bedeuten,
und ihre pharmakologisch verträglichen Salze mit Basen.

Bevorzugte Vertreter der Gruppe 3 sind solche der Formel I, worin einer der Substituenten R1 und R2 Methoxy, Difluormethoxy oder Ethoxy, und der andere 1-4C-Alkoxy, 4-6C-Cycloalkoxy, 3-6C-Cycloalkylmethoxy oder 1-2C-Polyfluoralkoxy bedeutet und X Cyanamino bedeutet, und ihre pharmakologisch verträglichen Salze mit Basen.

Bevorzugte Vertreter der Ausgestaltung 3a sind solche, in denen R2 1-4C-Alkoxy, 3-6C-Cycloalkylmethoxy oder 1-2C-Polyfluoralkoxy bedeutet.

Bevorzugte Vertreter der Ausgestaltung 3b sind solche, in denen R1 2-4C-Alkoxy, 4-6C-Cycloalkoxy, 3-6C-Cycloalkylmethoxy oder 1-2C-Polyfluoralkoxy bedeutet.

Die Ausgestaltung 3b ist gegenüber der Ausgestaltung 3a bevorzugt.

Besonders bevorzugte Vertreter der Gruppe 3 sind solche der Formel I, worin R1 2-4C-Alkoxy, Cyclopentyloxy, Cyclopropylmethoxy, Cyclobutylmethoxy, Difluormethoxy oder 2,2,2-Trifluorethoxy bedeutet, R2 Methoxy, Ethoxy oder Difluormethoxy bedeutet und X Cyanamino bedeutet, und ihre pharmakologisch verträglichen Salze mit Basen.

Die Verbindungen der Formel I können als tautomere Formen vorliegen. Das Proton der Hydroxygruppe bzw. der Cyanaminogruppe in 3-Stellung des Pyridinringes vermag zwischen diesen Gruppen und dem Stickstoff in 2-Stellung des Pyridazinrings zu wandern. Erfindungsgemäß ist bei Angabe oder Darstellung nur eines Tautomeren jeweils auch das andere Tautomere zu verstehen.

Die Verbindungen der Formel I sind bekannt aus den europäischen Patentanmeldungen bzw. Patenten EP 125 636, EP 163 965 und EP 393 500 bzw. sie können auf analoge Weise wie dort beschrieben hergestellt werden.

## Patentansprüche

1. Verwendung von Verbindungen der Formel I 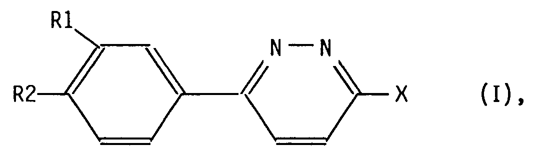 worin einer der Substituenten R1 und R2 Wasserstoff, 1-5C-Alkoxy oder Difluormethoxy, und der andere 1-5C-Alkoxy, 4-7C-Cycloalkoxy, 3-7C-Cycloalkylmethoxy, 3-5C-Alkenyloxy oder 1-4C-Polyfluoralkoxy bedeutet, X Hydroxy oder Cyanamino bedeutet, und ihren pharmakologisch verträglichen Salzen mit Basen zur Herstellung von Arzneimitteln für die Behandlung von Dermatosen.

2. Verwendung gemäß Anspruch 1 von Verbindungen der Formel I nach Anspruch 1, worin einer der Substituenten R1 oder R2 Methoxy und der andere 1-5C-Alkoxy oder 3-5C-Alkenyloxy und X Hydroxy bedeutet, und ihren pharmakologisch verträglichen Salzen mit Basen.

3. Verwendung gemäß Anspruch 1 von Verbindungen der Formel I nach Anspruch 1, worin einer der Substituenten R1 und R2 Wasserstoff oder 1-5C-Alkoxy und der andere 1-4C-Polyfluoralkoxy und X Hydroxy bedeutet, und ihren pharmakologisch verträglichen Salzen mit Basen.

4. Verwendung gemäß Anspruch 1 von Verbindungen der Formel I nach Anspruch 1, worin einer der Substituenten R1 und R2 Methoxy, Difluormethoxy oder Ethoxy, und der andere 1-5C-Alkoxy, 4-7C-Cycloalkoxy, 3-7C-Cycloalkylmethoxy, 3-5C-Alkenyloxy oder 1-4C-Polyfluoralkoxy und X Cyanamino bedeutet, und ihren pharmakologisch verträglichen Salzen mit Basen.

5. Verwendung gemäß Anspruch 1 von Verbindungen der Formel I nach Anspruch 1, worin R1 2-4C-Alkoxy, Cyclopentyloxy, Cyclopropylmethoxy, Cyclobutylmethoxy, Difluormethoxy oder 2,2,2-Trifluorethoxy bedeutet, R2 Methoxy, Ethoxy oder Difluormethoxy bedeutet und X Cyanamino bedeutet, und ihren pharmakologisch verträglichen Salzen mit Basen.

6. Verwendung gemäß Anspruch 1 einer Verbindung ausgewählt aus der Gruppe bestehend aus
6-(3-Methoxy-4-n-propoxyphenyl)-3[2H]pyridazinon,
6-(4-Isobutoxy-3-methoxyphenyl)-3[2H]-pyridazinon,
6-(3-Isobutoxy-4-methoxyphenyl)-3[2H]pyridazinon,
6-(4-Methoxy-3-n-propoxyphenyl)-3[2H]pyridazinon und
6-(4-Difluormethoxy-3-methoxyphenyl)-3[2H]pyridazinon
und den pharmakologisch verträglichen Salzen dieser Verbindungen.

7. Verwendung von Verbindungen der Formel I und ihren pharmakologisch verträglichen Salzen zu Behandlungen von Dermatosen bei Säugern, insbesondere Menschen.

8. Verwendung gemäß Anspruch 1 oder 2 oder 3 oder 4 oder 5 oder 6 oder 7 von Verbindungen der Formel I und ihren pharmakologisch verträglichen Salzen, dadurch gekennzeichnet, daß es sich bei den zu behandelnden Dermatosen um proliferative, entzündliche oder allergische Hauterkrankungen handelt.

9. Verwendung gemäß Anspruch 1 oder 2 oder 3 oder 4 oder 5 oder 6 oder 7 von Verbindungen der Formel I und ihren pharmakologisch verträglichen Salzen, dadurch gekennzeichnet, daß es sich bei den zu behandelnden Dermatosen um Psoriasis oder atopische Dermatitis handelt.
